# EUROPEAN PATENT APPLICATION

(11) **EP 4 656 177 A1**
(43) Date of publication of application: **03.12.2025**
(21) Application number: 24747489.3
(22) Date of filing: 26.01.2024
(51) Int. Cl.: A61K 8/64, A61K 8/98, A61Q 19/00, A61Q 19/02, A61Q 19/08, C12N 5/071

(54) **COMPOSITION CONTAINING NIDOGEN FOR IMPROVING SKIN CONDITION, TREATING SKIN DAMAGE OR MAINTAINING STEM CELL FUNCTION**

(30) Priority: 27.01.2023 KR 20230011151; 23.06.2023 KR 20230081156
(71) Applicant: Sungkwang Medical Foundation, Seoul 06135 (KR)
(72) Inventor: CHO, Eun-Gyung, Yongin-si, Gyeonggi-do 17008 (KR); LEE, Jin Hee, Seongnam-si, Gyeonggi-do 13634 (KR)
(74) Representative: AWA Sweden AB
(86) International application number: PCT/KR2024/001297
(87) International publication number: WO 2024/158256

(57) **Abstract**

The present invention relates to a composition for skin improvement, skin damage treatment, or maintenance of epidermal stemness, the composition comprising nidogen as an active ingredient. The composition comprising nidogen as an active ingredient according to an aspect exhibits effects on the epidermis and the dermis through basement membrane strengthening such as strengthening of binding between the basement membrane and the extracellular matrix so as to improve the function of the basement membrane, and proliferating of epidermal progenitor cells and maintaining of stemness of epidermal stem cells so as to improve keratin turnover and promote collagen production and binding, and thus the present invention can be utilized for skin improvement including skin barrier strengthening, suppression or prevention of hyperpigmentation, moisturization, skin elasticity, anti-aging, or tissue regeneration through wound healing.

## Description

### Technical Field

The present invention relates to a composition for improving the skin, treating skin damage, or maintaining epidermal stemness, comprising nidogen as an active ingredient.

### Background Art

The skin is composed of the epidermis, the dermis, and the subcutaneous fat layer in the stated order. The epidermis is divided into the stratum corneum, the stratum granulosum, the stratum spinosum, and the basal layer, in order from the outermost layer. Cells of the epidermis serve as bricks, and intercellular lipids between epidermal cells form a skin barrier. In addition, the epidermal cells of healthy people comprise natural moisturizing factors (NMFs) at high concentrations, which help retain moisture in the skin. For example, substances such as amino acids are water-soluble, and thus effectively bind with moisture and prevent the skin from drying out.

The stratum corneum of the skin is the outermost layer of the skin, and since it is in direct contact with the external environment, it plays an important barrier function in protecting the body from external physical and chemical stress. Such a barrier function is maintained by epidermal homeostasis. The epidermal homeostasis is the process by which keratinocytes in the basal layer undergo growth and division, and differentiation through cell migration, eventually reaching terminal differentiation and forming the skin barrier known as the stratum corneum, thereby maintaining continuous skin barrier functions (Korean J.Food.Sci. Technol. 43:458-463, 2011).

Nidogen is one of the important components constituting the basement membrane, along with type IV collagen, laminin, and heparan sulfate proteoglycan 2 (perlecan). Nidogen mediates binding between laminin and type IV collagen, and is accordingly involved in forming a three-dimensional structure which is a characteristic of the basement membrane. In addition to such a structural function of nidogen, nidogen has also been reported to be involved in cell attachment, neutrophil chemotaxis, and nerve development.

Accordingly, the inventors of the present invention confirmed that, when nidogen or both nidogen and an epidermal progenitor cell-conditioned medium were treated together, skin improvement effects, skin damage treatment effects, and epidermal stemness maintenance effects were observed, thereby completing the present invention.

### Description of Embodiments

### Technical Problem

One aspect is to provide a cosmetic composition, a composition for external preparation on skin, or a pharmaceutical composition, for skin improvement, treatment of skin damage, or maintenance of epidermal stemness, each comprising a nidogen protein as an active ingredient.

Another aspect is to provide a medium composition for maintenance of epidermal stemness, comprising a nidogen protein as an active ingredient.

Another aspect is to provide artificial skin including: a dermis including a fibroblast; and an epidermis including a keratinocyte and a nidogen protein.

Another aspect is to provide a preparation method for artificial skin, including: preparing a dermis by culturing a fibroblast; applying a nidogen protein to the dermis; and preparing an epidermis by applying a keratinocyte to the dermis to which the nidogen protein has been applied.

Another aspect is to provide a method of maintaining epidermal stemness of skin cells, including culturing skin cells in a medium comprising an isolated nidogen protein.

Another aspect is to provide a method of improving a skin condition or preventing, ameliorating, or treating skin damage, the method including administering an effective amount of a nidogen protein to a subject in need thereof.

Another aspect is to provide use of a nidogen protein for the manufacture of a preparation for improvement of a skin condition or prevention, amelioration, or treatment of skin damage.

### Solution to Problem

One aspect provides a composition comprising nidogen as an active ingredient.

In an embodiment, the composition may be a cosmetic composition, a composition for external preparation on skin, a pharmaceutical composition, or a medium composition.

In the present specification, "nidogen" refers to one of extracellular matrix proteins of a basement membrane found beneath epithelial cells in a living organism, is also called entactin, and interacts with laminin and collagen and serves as a bridge among basement membrane components of the skin. The nidogen may affect the basement membrane, the epidermis, or the dermis. The nidogen may be nidogen-1 or nidogen-2.

The nidogen may be a wild-type nidogen protein or a recombinant nidogen protein. Specifically, the recombinant nidogen protein may include a fragment of the wild-type nidogen protein, or may include a protein in which some amino acids included in the wild-type nidogen protein are substituted with other amino acids.

In an embodiment, the nidogen protein may include an amino acid sequence of SEQ ID NO: 1, or a sequence having at least about 95 %, at least about 97 %, at least about 98 %, or at least about 99 % sequence identity to the amino acid sequence of SEQ ID NO: 1.

In an embodiment, the composition may further include an epidermal progenitor cell-conditioned medium (EPC-CM).

The skin consists of the epidermis, the dermis, and the subcutaneous layer. The basal layer is located at the deepest part of the epidermis, and new cells constituting the epidermis (e.g., dermal cells or keratinocytes) are formed in this basal layer through cell division, and the formed cells that constitute the epidermis continuously replace dead cells in the upper part of the epidermis. This process induces regeneration of skin cells. The epidermal progenitor cells may be, for example, cells constituting the basal layer, and may be used interchangeably with the term "epidermal stem cells". The epidermal progenitor cells may be precursor cells of keratinocytes.

The epidermal progenitor cells may be derived from the stem cells. The epidermal progenitor cells may be cells of which differentiation and self-renewal ability are restricted compared to the stem cells. For example, the epidermal progenitor cells derived from the stem cells may be cells that have converted from cells having 100 % differentiation ability or arbitrary % differentiation ability to cells having 0 % differentiation ability or % differentiation ability lower than the arbitrary %. The epidermal progenitor cells derived from the stem cells may be used interchangeably with epidermal stem cells, differentiated epidermal stem cells, differentiated epidermal progenitor cells, or differentiated keratinocytes.

The epidermal progenitor cells may have increased expression of one or more selected from the group consisting of keratin 5 (KRT5), keratin 1 (KRT1), and keratin 14 (KRT14), compared to stem cells before culturing in a differentiation medium. That is, when the stem cells differentiate into epidermal progenitor cells, the expression of one or more selected from the group consisting of KRT5, KRT1, and KRT14 may increase. The epidermal progenitor cells derived from the stem cells may have a specific circular shape of a certain size.

The EPC-CM is a conditioned medium obtained after culturing differentiated epidermal progenitor cells in a medium, and may comprise a protein secreted outside the cell through interactions between cells during a culturing process of differentiated epidermal progenitor cells derived from the stem cells. The protein may include a useful protein such as a cytokine, a growth factor, and the like. The EPC-CM may comprise a number of useful proteins at a high concentration in the culture medium, and may include a common medium used in the art suitable for culturing cells. Specifically, the EPC-CM may include one or more selected from the group consisting of thrombospondin (TSP), a tissue inhibitor of metalloproteinases 1 (TIMP1), a tissue inhibitor of metalloproteinases 2 (TIMP2), ectodysplasin-A2 (EDA-A2) X-linked ectodysplasin-A receptor (XEDAR), angiopoietin-1, secreted protein acidic and rich in cysteine (SPARC), a transmembrane protein with EGF-like and two follistatin-like domains 1/tomoregulin-1 (TMEFF1/tomoregulin-1), nidogen-1, insulin-like growth factor-binding protein-3 (IGFBP-3), thrombospondin-2, tumor necrosis factor (TNF)-related activation-induced cytokine (TRANCE), and interleukin-15 receptor alpha (IL-15R alpha).

A culture of the epidermal progenitor cells may refer to a medium, differentiated epidermal progenitor cells, or a mixture thereof, obtained during or after the process of culturing differentiated epidermal progenitor cells in a medium.

The composition comprising nidogen of the present disclosure as an effective ingredient or the culture in a nidogen-coated plate was confirmed to exhibit effects on skin improvement by enhancing the adhesion ability or proliferation ability of keratinocytes in a concentration-dependent manner. In addition, a synergistic effect at treatment together with the EPC-CM or other extracellular matrix proteins (specifically, collagen) was confirmed to be achieved. Specifically, the composition comprising nidogen as an effective ingredient may improve the epidermis and keratin turnover by promoting proliferation and differentiation of epidermal precursor cells adjacent to the basement membrane by strengthening the basement membrane and the function induced thereby, may regulate pigment production by controlling the activity of melanin-forming cells, may act as a support to which a dermal collagen bundle may be fixed, and may promote collagen production in the dermis when treated together with the EPC-CM. Therefore, the composition comprising nidogen as an active ingredient was confirmed to exhibit an effect of improving the skin or maintaining epidermal stemness.

The composition comprising nidogen as an active ingredient or the culturing in a nidogen-coated plate may be used not only for attachment or proliferation of cells, but also for attachment or proliferation of artificial skin consisting of the cells, organoids, tissues, organs, and embryo, to a specific substrate or site. Specifically, effects of promoting engraftment of the artificial skin, the organoids, the tissues, and the organs to a specific substrate or site may be exhibited by pre-coating or treatment combination.

In an embodiment, the cosmetic composition may be for skin improvement or for maintenance of epidermal stemness.

In the present specification, the term "improvement" as used herein refers to any action that at least reduces a parameter related to the condition being treated, for example, the severity of a symptom, by administration of the composition according to the present disclosure.

In the present specification, the term "prevention" refers to any action that inhibits or delays the onset of a disease by administration of the composition.

The skin improvement may include one or more selected from the group consisting of skin barrier improvement, amelioration of hyperpigmentation, prevention or amelioration of skin wrinkles, skin moisturization, skin anti-aging enhancement, skin defense enhancement, skin elasticity enhancement, skin soothing, prevention or amelioration of skin damage, and prevention or amelioration of a skin disease.

The skin improvement may include skin regeneration or skin protection from external stimuli. The term "skin regeneration" may be used interchangeably with the terms "skin recovery," "skin reconstruction," or "skin repair."

The external stimuli may include ultraviolet (UV) rays, scratches, friction, trauma, inflammation, heat, temperature, acids, bases, surfactants, toxic substances, viral infections, fine dust, etc., but is not limited thereto, and may include any form of physical or chemical stimulation that can cause skin damage. By the external stimuli, cells constituting the human epidermis (e.g., keratinocytes), cells constituting the dermis (e.g., fibroblasts), or the basement membrane may be damaged. The UV rays may be one or more of UVA, UVB and UVC.

The skin regeneration is a broad concept that includes the ability to produce new keratinocytes in response to general skin damage. The skin damage may include any type of skin wound, skin scar, or skin burn, caused by external physical or chemical stimuli. For example, the skin damage may include incised wounds, abrasions, puncture wounds, lacerations, ulcers, bedsores, contusions, and avulsions, but is not limited thereto.

The skin protection refers to suppression or prevention of skin damage as described above.

The nidogen may inhibit separation of the epidermis and the dermis caused by the external stimuli. More specifically, it may inhibit formation of blistering caused by separation of the epidermis and the dermis caused by the external stimuli.

The nidogen may recover the arrangement of keratinocytes in the basal layer and the structure of the basement membrane damaged by the external stimuli. More specifically, the nidogen may repair structural damage, such as hemidesmosomes at the dermo-epidermal junction, anchoring fibrils, and dermal collagen, caused by the external stimuli, or may prevent damage caused by the external stimuli.

The skin improvement may include inhibition or prevention of hyperpigmentation.

Melasma skin exhibits a series of structural and functional changes in the epidermis, the basement membrane, and the upper dermis, and these changes interact to induce and maintain a localized hypermelanogenic phenotype. In facial melasma, the basement membrane is thinned and discontinuous shape thereof is observed, and compared to the healthy skin, more disruption or gaps in the basement membrane are observed in the melasma skin. That is, damage to the basement membrane is related to melanogenesis in the melasma skin and the photoaged skin. In the present disclosure, the nidogen as defined above may induce maintenance of the structure of the basement membrane and bonding between an extracellular matrix and the basement membrane, such that, through melanin production in the skin induced with melasma skin or photoaging and adjustment of melanin-forming cell activity, pigmentation including melasma and/or blemishes may be improved and/or skin whitening effects may be induced. Accordingly, the nidogen of the present specification may be additionally provided to a composition for inhibiting melanogenesis and/or suppressing hyperpigmentation (or for whitening melasma- or photoaging-induced skin).

The nidogen may recover proliferation and differentiation of the epidermis due to the external stimuli. More specifically, the nidogen may alleviate a phenomenon of inhibition of proliferation of keratinocytes in the basal layer of the epidermis caused by the external stimuli, or may induce expression of Ki67. In addition, the nidogen may alleviate a phenomenon of inhibition of differentiation of keratinocytes in the upper layer of the epidermis, or may induce expression of filaggrin (FLG).

In an embodiment, the protein may improve binding between the extracellular matrix and the basement membrane, or the dermal density.

In an embodiment, the protein may promote production of collagen in the dermis or Ki67, KRT14, FLG in the epidermis.

In the present specification, the term "skin barrier improvement" refers to both skin barrier strengthening or protection function, wherein the skin barrier is the outermost layer of the epidermis, called the stratum corneum, which is mainly composed of flat, nucleus-free corneocytes. The multi-lamellar lipid layer, formed by intercellular lipids such as ceramides, cholesterols, and fatty acids synthesized by keratinocytes of the skin barrier, which is maintained through a process of division and differentiation of cells that constitute the normal epidermis, may act as a protective barrier to prevent moisture from evaporating from the skin.

The skin barrier improvement may refer to any action that alleviates skin diseases caused by weakened skin barrier function, such as psoriasis, contact dermatitis, eczematous dermatitis, actinic dermatitis, seborrheic dermatitis, dermatitis herpetiformis, lichen planus, lichen sclerosis, pyoderma gangrenosum, pemphigus, epidermolysis bullosa, systemic sclerosis, or leprosy, or that improves damaged skin barrier function.

In the present specification, the term "prevention or amelioration of skin wrinkles" refers to prevention, inhibition, or suppression of the formation of wrinkles on the skin, or alleviation of wrinkles that have already formed.

In the present specification, the term "skin elasticity improvement effect" refers to prevention, inhibition, or suppression of the decline in skin elasticity, or amelioration of skin elasticity that has already declined.

In an embodiment, the protein may improve functions of the basement membrane. Specifically, the protein may improve binding between the basement membrane and the extracellular matrix or binding between the epidermis and the dermis, or may improve density of the dermis.

In an embodiment, the protein may promote production of collagen in the dermis or Ki67, KRT14, FLG in the epidermis.

In the present specification, the term "stemness" is a term commonly used in the art to collectively refer to pluripotency, which is the ability to produce all cells including embryonic stem cells, and self-renewal, which is the ability to indefinitely produce cells similar to oneself. That is, the stemness may refer to the ability to maintain the characteristics of the stem cells.

In the present specification, the term "maintenance of stem cell function" refers to characteristics of inhibiting cellular aging of the stem cells, increasing cell proliferation ability of the stem cells, increasing functionality of the stem cells, increasing telomerase activity of the stem cells, increasing expression of stem cell factors, proliferating undifferentiated cells while maintaining the undifferentiated state, increasing protein homeostasis in the stem cells, or increasing cell migration activity.

The term "stem cell factor" refers to a gene highly expressed in the stem cells and known to play a crucial role in maintaining pluripotency of the stem cells, i.e., the characteristics of the stem cells, and examples of the gene are SOX2, OCT4, NANOG, KLF4, C-MYC, Oct3/4, SSEA-1, SSEA-3, SSEA-4, TRA1-60, TRA1-81, Lin28, Fbx15, and the like, but are not limited thereto. Therefore, when the expression of at least one of the stem cell factors is observed, the stem cells may be considered undifferentiated stem cells, and the higher the expression of these factors, the higher the pluripotency of the stem cell may be determined.

The term "increased functionality" refers to functional maturation, and is associated with a gene known to be expressed in stem cells and play an important role in maintaining the mature functional characteristics of the stem cells, and examples of the gene are CYP3A4, CYP3A7, ALB, AFP, TTR, CK19, and the like, but are not limited thereto. Therefore, when the expression of at least one of the factors that increase the functionality of the stem cells is observed, it may be determined that the functionality of the stem cells may be increased, and the more these factors are expressed, the higher the ability to increase the functionality of the stem cells.

The term "proliferation" refers to an increase in the number of cells, and may be used in the same meaning as the term "growth". In particular, the term "undifferentiated proliferation" refers to proliferation of the stem cells that have not differentiated into specific cells and retain the same properties, i.e., pluripotency (which may be used interchangeably with the term "totipotency"), as the original cells.

The term "culture medium" refers to a substance that supports the growth and survival of various cells, including stem cells and keratinocytes, during 2D or 3D culture in vitro, as well as during culturing of artificial skin, organoids, tissues, organs, or embryos.

In an embodiment, the nidogen may upregulate expression of a gene of the group consisting of Ki67, keratin 14 (KRT14), integrin alpha-6 (ITGA6), TP63, and SRY-box transcription factor 2 (SOX2). Specifically, the nidogen may be provided in the form of a medium composition or in a coated form such that expression of a gene or protein of the group consisting of Ki67, KRT14, ITGA6, TP63, and SOX2 of keratinocytes.

In an embodiment, it was confirmed that the nidogen inhibited aging of the stem cells when added to a culture medium for the stem cells, enhanced cell proliferation ability, and significantly increased stemness by inducing expression of proteins related to the stemness. Therefore, the protein may improve the turnover of the stratum corneum.

The composition may be provided in the form of a medium composition comprising the nidogen, or in a nidogen-coated form, for improving the skin (e.g., inhibiting epidermal-dermal separation, maintaining the basement membrane structure, alleviating inhibition of epidermal differentiation, and regulating proliferation, differentiation, and migration of melanin-forming cells in the basal layer of the epidermis), and/or for enhancing the stemness of the epidermal stem cells. Specifically, the nidogen may be provided in a form in which a plate for culturing cells is coated with the nidogen , or in a form in which there is nidogen coating between the epidermis and dermis in artificial skin. The nidogen may be added to a common medium used for culturing the stem cells to achieve the effect related to enhancing the stemness disclosed in the present specification.

The nidogen may be included in an amount of 0.00001 to 90 wt% based on a total weight of the cosmetic composition. For example, the amount may be 0.00001 to 80 wt%, 0.00001 to 70 wt%, 0.00001 to 60 wt%, 0.00001 to 50 wt%, 0.00001 to 40 wt%, 0.00001 to 30 wt%, 0.00001 to 20 wt%, 0.00001 to 10 wt%, 0.00001 to 5 wt%, 0.00001 to 3 wt%, 0.00001 to 1 wt%, 0.0001 to 90 wt%, 0.0001 to 70 wt%, 0.0001 to 60 wt%, 0.0001 to 50 wt%, 0.0001 to 40 wt%, 0.0001 to 30 wt%, 0.0001 to 20 wt%, 0.0001 to 10 wt%, 0.001 to 80 wt%, 0.001 to 70 wt%, 0.001 to 60 wt%, 0.001 to 50 wt%, 0.001 to 40 wt%, 0.001 to 30 wt%, 0.001 to 20 wt%, 0.001 to 10 wt%, 0.01 to 10 wt%, 0.01 to 5 wt%, 0.01 to 3 wt%, 0.01 to 1 wt%, 0.1 to 10 wt%, 0.1 to 5 wt%, 0.1 to 3 wt%, 0.1 to 1 wt%. Additionally, the composition may further comprise an EPC-CM.

The cosmetic composition may be prepared in any formulation commonly prepared in the technical field to which the present invention belongs. For example, it may be prepared in the form of a face lotion (skin lotion), a skin product, a skin softener, a skin toner, a skin booster, an astringent, a lotion, a milk lotion, a moisture lotion, a nourishing lotion, a serum, a massage cream, a nourishing cream, a moisture cream, a hand cream, a hand sanitizer, a foundation, an essence, a nourishing essence, a pack, a mascara, a soap, a foam cleansing, a cleansing lotion, a cleansing cream, a body lotion, a body cleanser, a suspension, a gel, a powder, a paste, a mask pack, a sheet, a stick product, a balm-type product, a spray, a freeze-dried product, a powder, a syringe, or a pre-filled syringe. The compositions of such formulations may be prepared according to methods conventional in the art. A mixing amount of additional ingredients such as the moisturizer may be easily selected by those skilled in the art within a range that does not impair the purpose and effect of the present invention.

The cosmetic composition may additionally comprise functional additives and ingredients included in general cosmetic compositions, in addition to the effective ingredients disclosed in the present specification, and may further contain purified water, a thickener, a preservative, a stabilizer, a solubilizer, a surfactant, a carrier, a fragrance, or a combination thereof that are commonly used. The functional additive may include an ingredient selected from the group consisting of water-soluble vitamins, essential vitamins, polymeric peptides, polysaccharide polymers, sphingolipids, and seaweed extracts. Examples of the carrier are alcohol, oils, surfactants, fatty acids, silicone oils, humectants, moisturizers, viscosity modifiers, emulsifiers, stabilizers, UV scatterers, UV absorbers, coloring agents, and fragrances. Compounds/compositions that may be used as alcohol, oil, a surfactant, a fatty acid, silicone oil, a humectant, a moisturizer, a viscosity modifier, an emulsifier, a stabilizer, a UV scatterer, a UV absorber, a coloring agent, and a fragrance are already known in the art, and thus those skilled in the art may select and use appropriate substances/compositions. In addition, the cosmetic composition may further comprise, as needed, other ingredients such as a UV blocking agent, an antioxidant (e.g., butyl hydroxyanisole, propyl gallate, erythorbic acid, tocopheryl acetate, butylated hydroxytoluene, etc.), a preservative (e.g., methylparaben, butylparaben, propylparaben, phenoxyethanol, imidazolidinyl urea, chlorphenesin, etc.), a coloring agent, a pH adjuster (e.g., triethanolamine, citric acid, citric acid sodium, malic acid, malic acid sodium, fumaric acid, fumaric acid sodium, succinic acid, succinic acid sodium, sodium hydroxide, sodium monohydrogen phosphate, etc.), a moisturizer (e.g., glycerin, sorbitol, propylene glycol, butylene glycol, hexylene glycol, diglycerin, betaine, glycereth-26, methyl gluceth-20, etc.), a lubricant.

In addition, regarding the cosmetic composition of each formulation, appropriate ingredients may be selected and formulated according to the formulation or intended use of the cosmetics. The formulation ingredients and methods are known in the art, and are not described herein in detail.

Another aspect provides a composition for external preparation on skin or a pharmaceutical composition, each comprising nidogen as an active ingredient.

In an embodiment, the composition may further comprise an EPC-CM.

In an embodiment, the external preparation on skin may be for skin improvement, maintenance of epidermal stemness, or wound treatment and tissue repair.

The terms "nidogen," "EPC-CM," "skin improvement," and "epidermal stemness" are as defined above.

The nidogen may be included in an amount of 0.00001 to 90 wt% based on the total weight of the composition for external preparation on skin. For example, the amount may be from 0.00001 to 80 wt%, 0.00001 to 70 wt%, 0.00001 to 60 wt%, 0.00001 to 50 wt%, 0.00001 to 40 wt%, 0.00001 to 30 wt%, 0.00001 to 20 wt%, 0.00001 to 10 wt%, 0.00001 to 5 wt%, 0.00001 to 3 wt%, 0.00001 to 1 wt%, 0.0001 to 90 wt%, 0.0001 to 70 wt%, 0.0001 to 60 wt%, 0.0001 to 50 wt%, 0.0001 to 40 wt%, 0.0001 to 30 wt%, 0.0001 to 20 wt%, 0.0001 to 10 wt%, 0.001 to 80 wt%, 0.001 to 70 wt%, 0.001 to 60 wt%, 0.001 to 50 wt%, 0.001 to 40 wt%, 0.001 to 30 wt%, 0.001 to 20 wt%, 0.001 to 10 wt%, 0.01 to 10 wt%, 0.01 to 5 wt%, 0.01 to 3 wt%, 0.01 to 1 wt%, 0.1 to 10 wt%, 0.1 to 5 wt%, 0.1 to 3 wt%, or 0.1 to 1 wt%. In addition, the composition may further comprise the EPC-CM.

The external preparation on skin may be formulated in any form including cream, gel, ointment, foam, spray, powder, freeze-dried type, liquid, skin emulsifier, skin suspension, transdermal patch, drug-containing bandage, lotion, serum, roll-on type, paste, balsam, stick, balm, syringe, pre-filled syringe, and the like. The external preparation on skin may be appropriately formulated, as needed, with ingredients commonly used in external preparation on skin such as cosmetics, pharmaceuticals, and medical devices, including water-based ingredients, oil-based ingredients, powdered ingredients, alcohols, moisturizers, thickeners, UV absorbers, whitening agents, preservatives, antioxidants, surfactants, fragrances, coloring agents, various skin nutrients, or a combination thereof. The external preparation on skin may be appropriately formulated with chelating agents, such as disodium edetate, trisodium edetate, sodium citrate, sodium polyphosphate, sodium metaphosphate, gluconic acid, caffeine, tannin, verapamil, a licorice extract, glabridin, a hot water extract of fruits of Calin, various herbal medicines, tocopheryl acetate, glycyrrhizic acid, tranexamic acid a derivative or salt thereof, vitamin C, magnesium ascorbyl phosphate, ascorbic acid glucoside, arbutin, Kojic acid, or sugars, such as glucose, fructose, or treharose, natural polymer materials, such as alginate, chitosan, collagen, hyaluronic acid, etc., and synthetic polymer materials, such as poly(vinyl alcohol) (PVA), poly(N-vinyl-2-pyrrolidone) (PVP), poly(ethylene glycol) (PEG), carboxymethyl cellulose (CMC). etc.

The skin includes all skin areas of the body, including a face, hands, arms, legs, feet, chest, abdomen, back, buttocks, and scalp.

Another aspect provides a method of maintaining the epidermal stemness of skin cells, the method including culturing skin cells in a medium comprising an isolated nidogen protein.

Another aspect provides a method of improving a skin condition or preventing, ameliorating, or treating skin damage, the method including administering an effective amount of a nidogen protein to a subject in need thereof.

Another aspect provides use of a nidogen protein for the manufacture of a preparation for improvement of a skin condition or prevention, amelioration, or treatment of skin damage

Another aspect provides artificial skin including a nidogen protein.

The nidogen protein is as defined above.

In the present specification, the term "artificial skin" refers to a three-dimensional reconstruction of skin using skin cells and skin components, and is used in its broadest sense to include all polymer composites that exhibit structural and functional characteristics similar to those of actual skin that can be used by those skilled in the art. Artificial skin may be composed of a dermis and an epidermis, similar to the human skin. In the present specification, the artificial skin may be composed of a dermis including fibroblasts and an epidermis including keratinocytes and a nidogen protein. For example, the artificial skin that has been described in Larouche et al. BioResearch Open Access (2016) 5(1):320-329 may be used, the artificial skin is not limited thereto.

In addition, the artificial skin may include a higher-level skin model that is possible to mimic the complexity and functions of skin tissue in vivo by further implementing hair follicles, sweat glands, sebaceous glands, melanocytes, blood vessels, nerve cells, or subcutaneous fats, in addition to the dermis and the epidermis. That is, in the present specification, the artificial skin may be used to encompass 'skin equivalents or reconstructed skin,' 'skin organoids,' or 'skin models.'

The dermis may further include an extracellular matrix, in terms of the correlation with the actual human skin. For example, the dermis may further include an extracellular matrix that constitutes the dermis, such as collagen, laminin, elastin, chitosan, glycosaminoglycans (GAGs), and hyaluronic acid (HA).

A thickness of the dermis may be generally 0.05 cm or more, specifically 0.05 cm to 2 cm, but is not limited thereto. The thickness may be increased or decreased as long as it does not impair the advantageous characteristics of the artificial skin according to the present invention.

The epidermis may be located on the dermis. The nidogen protein may exist in the basement membrane of the epidermis within the artificial skin, and may be provided in a coated form between the dermis and the epidermis.

The keratinocytes may be any commercially available keratinocytes, including not only keratinocytes directly isolated or keratinocytes directly isolated from humans and cultured, but also keratinocytes derived from other cells and commercially available.

Another aspect provides a preparation method for artificial skin, including: preparing a dermis by culturing a fibroblast; applying a nidogen protein to the dermis; and preparing an epidermis by applying a keratinocyte to the dermis to which the nidogen protein has been applied.

The nidogen protein is as defined above.

A concentration of the nidogen protein may be 0.1 ug/ml to 1000 ug/ml, 0.1 ug/ml to 500 ug/ml, 0.1 ug/ml to 200 ug/ml, 0.1 ug/ml to 150 ug/ml, 0.1 ug/ml to 100 ug/ml, 1 ug/ml to 1000 ug/ml, 1 ug/ml to 500 ug/ml, 1 ug/ml to 200 ug/ml, 10 ug/ml to 1000 ug/ml, 10 ug/ml to 500 ug/ml, 10 ug/ml to 200 ug/ml, 20 ug/ml to 1000 ug/ml, 30 ug/ml to 500 ug/ml, 40 ug/ml to 200 ug/ml, 45 ug/ml to 200 ug/ml, or 50 ug/ml to 200 ug/ml.

The preparing of the dermis by culturing a fibroblast may include culturing the fibroblast for about 1 week to 8 weeks, about 3 weeks to 5 weeks, or about 4 weeks after preparing the fibroblast.

The applying of the nidogen protein may additionally include drying the nidogen protein after applying the nidogen protein.

The preparing of the epidermis may include: manufacturing a dermal-epidermal structure by applying a keratinocyte; and then culturing the dermal-epidermal structure on a separate dermis.

The epidermis may be cultured in an epidermis-forming medium. The epidermis-forming medium may be any medium known in the art, and for example, may be a medium supplemented with bovine pituitary extract (BPE), human epidermal growth factor (hEGF), bovine insulin, hydrocortisone, and gentamicin and amphotericin -B(GA-1000). In addition, the medium may be further supplemented with epinephrine and transferrin in addition to the aforementioned ingredients.

The epidermis-forming medium is placed so that it contacts the lower surface of the dermis, and the opposite surface of the epidermis-forming medium may be exposed to air.

In an embodiment, the dermal-epidermal structure may be obtained by culturing the keratinocyte for 1 day to 14 days, 4 days to 11 days, 6 days to 8 days, or 7 days.

In an embodiment, the dermis may be obtained by culturing the dermal-epidermal structure on another dermis for 1 day to 30 days, 10 days to 20 days, 13 days to 15 days, or 14 days.

When the artificial skin is prepared by the preparation method, an artificial skin model substantially similar to the actual skin may be obtained, and the artificial skin may have improved characteristics by the nidogen protein.

### Advantageous Effects of Disclosure

A composition comprising nidogen as an effective ingredient according to an aspect exhibits effects on the epidermis and the dermis through basement membrane strengthening such as strengthening of the bond between the basement membrane so as to improve the function of the basement membrane and an extracellular matrix, and proliferating of epidermal progenitor cells and maintaining of stem cell function of epidermal stem cells so as to improve keratin turnover and promote collagen production and binding, and thus the present invention can be utilized for skin improvement including skin barrier strengthening, suppression or prevention of hyperpigmentation, moisturization, skin elasticity, anti-aging, or tissue regeneration through wound healing.

### Brief Description of Drawings

FIG. 1 is a graph analyzing cytotoxicity of nidogen in HEK and HaCaT cells.
FIG. 2 is an image confirming an effect of nidogen coating concentration on cell adhesion ability.
FIG. 3 is an image comparing cell adhesion ability according to coating with nidogen and other extracellular matrix proteins (gelatin and collagen).
FIG. 4 is an image comparing cell proliferation ability according to coating with nidogen and other extracellular matrix proteins (gelatin and collagen).
FIG. 5 is a graph comparing cell proliferation ability according to coating with nidogen and other extracellular matrix proteins (gelatin and collagen).
FIG. 6 shows an image (top) and a graph (bottom) of cell proliferation, showing a synergistic effect of double coating of nidogen and collagen on cell proliferation.
FIG. 7 is an image showing an epidermal-dermal adhesion effect of nidogen in artificial skin irradiated with UVB (20X Scale bar: 100 µm, 40X Scale bar: 50 µm).
FIG. 8 is an image showing a restorative effect of nidogen on a basement membrane structure in artificial skin irradiated with UVB.
FIG. 9 is an image showing a restorative effect of nidogen on epidermal differentiation in artificial skin irradiated with UVB (20X Scale bar: 100 µm, 40X Scale bar: 50 µm).
FIG. 10 is an image showing a restorative effect of nidogen on proliferation of a basal layer of the epidermis in artificial skin irradiated with UVB (20X Scale bar: 100 µm, 40X Scale bar: 50 µm).
FIG. 11 is an image showing a restorative effect of nidogen on collagen production in a dermis of artificial skin irradiated with UVB (20X Scale bar: 100 µm, 40X Scale bar: 50 µm).
FIG. 12 shows a western blot image (left) showing an increase in expression of Ki67 and KRT14, which are proteins related to stemness of cells upon nidogen coating, and a graph (right) quantifying intensity of each band after standardization.
FIG. 13 is a graph showing a synergistic effect of nidogen coating and an epidermal progenitor cell culture medium on cell proliferation.
FIG. 14 is a graph showing an effect of a formulation comprising nidogen and an epidermal precursor cell culture medium on cell proliferation.

### Mode for Invention

Hereinafter, preferable Examples are presented to help understanding of the present disclosure. However, the following examples are only presented for easier understanding of the present disclosure, and the contents of the present disclosure are not limited by the following examples. Examples can undergo various modifications, and thus examples are not limited to those disclosed below and can be implemented in various forms.

### Example 1. Preparation of nidogen-1 recombinant protein

An expression vector including nidogen-1 DNA sequences was manufactured. After transforming the nidogen-1 expression vector into E. *coli,* first, a strain including the expression vector was obtained first. Second, the strain that successfully expressed the nidogen-1 protein was screened, and finally, a stock of strains expressing the nidogen-1 protein was obtained. The strain was subjected to lab-scale culturing to confirm the expression and purification of the nidogen-1 protein, followed by large-scale production of 200 L. The cultured strain was collected through continuous centrifugation, and then crushed to obtain inclusion bodies. After dissolving the inclusion bodies, the nidogen-1 protein was purified using various columns suitable for protein characteristics in sequence. Finally, after concentration and buffer exchange, a high-concentration of nidogen-1 (hereinafter referred to as 'nidogen') protein substance was produced.

### Example 2. Preparation of epidermal progenitor cell-conditioned medium (EPC-CM)

### 2.1. Differentiation of stem cells into epidermal progenitor cells

Informed consent was received from healthy mothers who had given a normal birth, based on sufficient explanation, and the umbilical cord was separated from the placenta collected during normal placental delivery. The separated placenta and umbilical cord were each washed 2 to 5 times with Ca/Mg-free Dulbecco's Phosphate-Buffered Saline (DPBS) to remove blood. Afterwards, the placenta tissue was cut into a size of about 1 to 5 mm. In addition, after removing the arteries and veins from the umbilical cord, the umbilical cord was cut into a size of about 1 to 5 mm. Subsequently, the placenta and umbilical cord were each attached to a culture container and cultured for 10 to 15 days. After confirming that cells had begun to proliferate in the cultured tissue, 200 U/mL of collagenase I was added for 5 to 6 hours to separately isolate placenta-derived stem cells and umbilical cord-derived stem cells.

To confirm whether the placenta-derived stem cells exhibit the characteristics of mesenchymal stem cells, flow cytometry was performed for surface protein analysis. The cells were then washed with DPBS and added to DPBS containing 2 % FBS to proceed a reaction for about 20 minutes with CD44, CD73, CD90, CD105, CD45, CD34, CD31, CD29, CD49, CD9, HLA-ABC, and HLA-ER antibodies. Then, the surface antigen characteristics were analyzed using a flow cytometer (FACS Calibur, Becton Bickinson). From the high expression levels of CD44, CD73, CD90, CD105, CD29, CD49, CD9, and HLA-ABC in the placenta-derived stem cells, it was confirmed that the placenta-derived stem cells had the characteristics of mesenchymal stem cells.

The isolated cells were inoculated into a multi-flask at a concentration of 100 to 5,000 cell/cm², the cells, referred to as P0, were sub-cultured for 5 passages for 2 days in an MEM alpha GlutaMAX (PS-CM) medium supplemented with 25 ng/ml of fibroblast growth factor-4 (FGF-4), 1 µg/ml of heparin, 50 µg/ml of gentamicin, and 10 % fetal bovine serum (FBS) under conditions of 37 °C and 5 % CO₂.

Afterwards, the cultured cells were added to a multi-flask at 5 mℓ/cm² containing a Dulbecco's Modified Eagle Medium (DMEM/F12, Nutrient Mixture F-12) and a differentiation medium supplemented with 0.3 µm of ascorbic acid, 0.5 µg/ml of hydrocortison, and 10 % of FBS, and then cultured for 11 days under conditions of 37 °C and 5 % CO₂. As a result, the placenta-derived stem cells and the umbilical cord-derived stem cells differentiated into small, uniform, round cells similar to epidermal progenitor cells in the differentiation medium.

### 2.2. Preparation of epidermal progenitor cell-conditioned medium

A medium was produced from epidermal progenitor cells differentiated in Example 2.1. The differentiated epidermal progenitor cells were removed from the differentiation medium and washed with DPBS to remove residual serum. Afterwards, a DMEM/F12 medium not containing choline chloride and phenol red was added to a culture plate at a concentration of 2 to 3 mℓ/cm², followed by culturing for 3 days under conditions of 37 °C and 5 % CO₂. Subsequently, the supernatant was collected from the culture of the differentiated epidermal progenitor cells and epidermal progenitor cells mixed with the medium. Next, the supernatant was filtered through a 0.22 µm filter to obtain stem cell-derived EPC-CM.

### Experimental Example 1. Cytotoxicity evaluation after nidogen treatment

The cytotoxicity of the nidogen-1 protein was evaluated using human epidermal keratinocytes(HEKs) or HaCaT which is a human keratinocyte cell line.

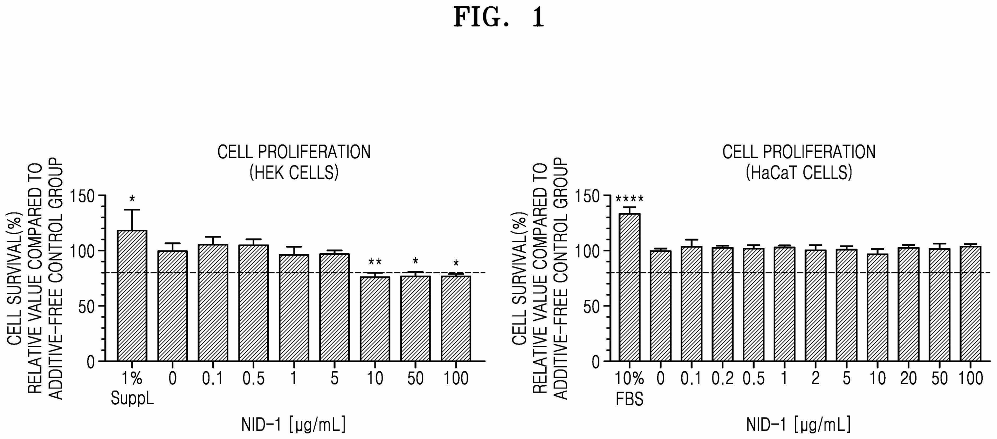

Specifically, HEKs or HaCaT cells (3 x 10⁴ cells/well) were dispensed into a 48-well plate, and then cultured in EpiLife (Gibco, USA) with HKGS supplement (Gibco, USA) or DMEM (WELGENE, Korea) with 10 % FBS (Gibco, USA) for 24 hours. After replacing the medium with a supplement-free medium or a serum-free medium, cells were cultured for 6 hours and 24 hours, respectively, treated with the nidogen protein at various concentrations (1 to 100 mg/mL), and cultured for additional 24 hours. The cytotoxicity was then evaluated using EZ-Cytox (DoGenBio, Korea). EZ-Cytox equivalent to 1/10 of the medium volume per well was added and maintained at 37 °C in a CO₂ incubator for 30 minutes. The absorbance was measured at 450 nm, and the results are shown in FIG. 1.

FIG. 1 is a graph analyzing the cytotoxicity of nidogen in the HEK cells and HaCaT cells.

As shown in FIG. 1, the HEK cells did not exhibit toxicity when treated with the nidogen protein at a concentration of less than 10 µg/mL, and the HaCaT cells did not exhibit toxicity regardless of the concentration of the nidogen treatment.

### Experimental Example 2. Evaluation of cell adhesion and survival rate after nidogen coating

### 2.1. Coating effect of nidogen protein on adhesion of human keratinocytes

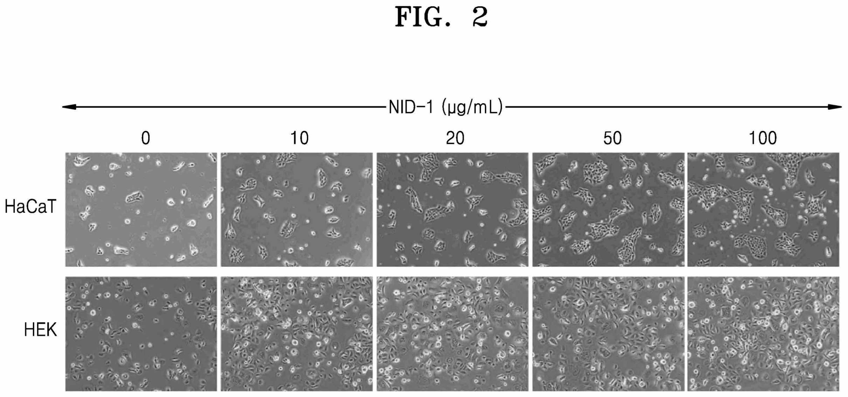

The nidogen protein quantified using the Pierce BCA protein assay kit (Thermo Scientific, USA) was diluted in DPBS at different concentrations (0, 10, 20, 50, and 100 µg/ml). A coverslip was placed in a 24-well plate, and 500 µI of the nidogen dilution was dispensed into each well for coating at 4 °C for 24 hours. Next day, 500 µI of 0.5 % BSA solution was dispensed into each well, followed by incubation at room temperature for 1 hour and washing with DPBS. The HaCaT or HEK cells were suspended in serum-free DMEM or 1 % supplement-containing EpiLife medium, and then seeded at 5 x 10⁴ cells per well and cultured for 24 hours at 37 °C in a CO₂ incubator. After 24 hours, images of the cells were captured, and the results are shown in FIG. 2.

FIG. 2 is an image confirming the effect of nidogen coating concentration on cell adhesion ability.

As shown in FIG. 2, it was confirmed that the adhesion ability of two types of keratinocytes (HEK cells and HaCaT cells) was improved in a concentration-dependent manner with the nidogen coating.

### 2.2. Comparison of coating effect on nidogen protein and other extracellular matrix proteins in adhesion of human keratinocytes

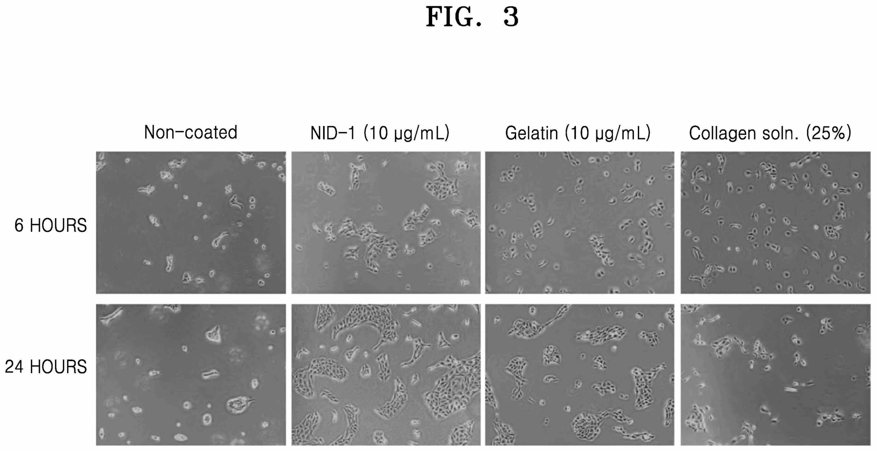

A non-treated cell culture plate (24-well plate)(NEST Scientific, China) was coated at 4 °C for 24 hours with 10 µg/mL of nidogen-1, 10 µg/mL of gelatin, and 25 % collagen solution (collagen concentration, 12.5 µg/mL) dispensed thereto. Next day, 500 µI of 0.5 % BSA solution was dispensed into each well, followed by incubation at room temperature for 1 hour and washing with DPBS. The HaCaT cells were suspended in serum-free DMEM and seeded at 5 x 10⁴ cells per well. The cells were then cultured for 6 hours and 24 hours at 37 °C in a CO₂ incubator. After 6 hours and 24 hours of culture, images of the cells were captured, and the results are shown in FIG. 3.

FIG. 3 is an image comparing the cell adhesion ability according to coating with nidogen and other extracellular matrix proteins (gelatin and collagen).

As shown in FIG. 3, the cells attached to the nidogen-coated plate formed colonies resembling undifferentiated keratinocytes and proliferated, whereas the cells attached to gelatin- or collagen-coated plate spread widely and proliferated.

### 2.3. Coating effect of nidogen protein on proliferation of human keratinocytes

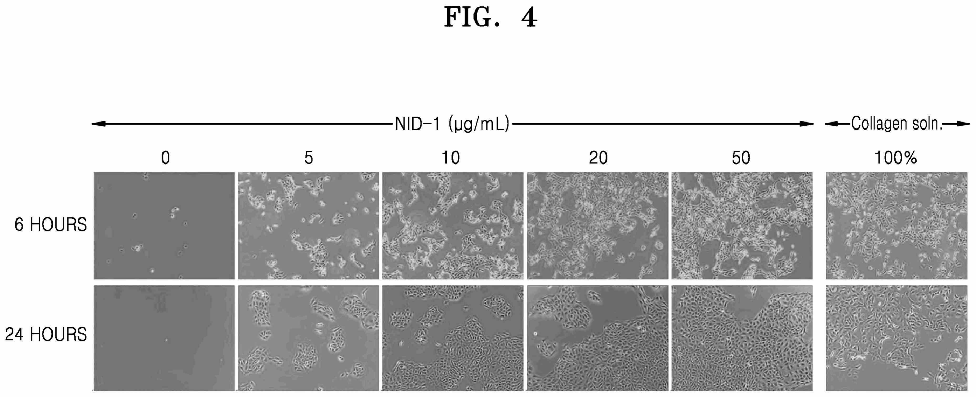

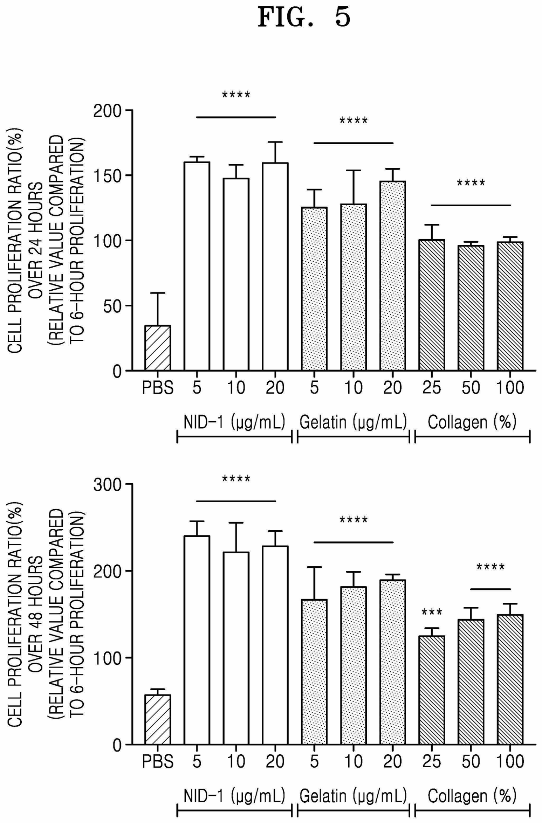

The nidogen protein quantified using the Pierce BCA assay kit was diluted with DPBS at different concentrations (0, 5, 10, 20, and 50 µg/ml) and dispensed into a non-treated cell culture plate (e.g., a 24-well plate) at 500 µI per well for coating at 4 °C for 24 hours. Gelatin (5, 10, and 20 µg/ml) and collagen solution(25 %, 50 %, and 100 %) were also diluted and used for coating in the same manner. Next day, 500 µl of 0.5 % BSA solution was dispensed into each well, followed by incubation at room temperature for 1 hour and washing with DPBS. The HaCaT cells as a human keratinocyte cell line were suspended in serum-free DMEM and seeded at 5 x 10⁴ cells per well. The cells were then cultured for 6 hours at 37 °C in a CO₂ incubator to allow adhesion to the dishes. After 6 hours, non-adherent cells were washed away with DPBS, and the remaining adherent cells were cultured in serum-free DMEM medium for 24 hours and 48 hours. Images of the cells were taken at 6, 24, and 48 hours after seeding, and the results are shown in FIG. 4. The cell survival and proliferation were measured by adding EZ-Cytox reagent (DoGenBio) equivalent to 1/10 of the medium volume per well after obtaining images at each time point. Following incubation for 30 minutes at 37 °C in a CO₂ incubator, the absorbance at a wavelength of 450 nm was measured, and the results are shown in FIG. 5. The cell proliferation rate at 24 hours and 48 hours was calculated using the following equation : [(absorbance at 24 or 48 h - absorbance at 6 h)/absorbance at 6 h] x 100.

FIG. 4 is an image comparing the cell proliferation ability according to coating with nidogen and other extracellular matrix proteins (gelatin and collagen).

FIG. 5 is a graph comparing the cell proliferation ability according to coating with nidogen and other extracellular matrix proteins (gelatin and collagen).

As shown in FIGS. 4 and 5, the cells in the nidogen protein-coated plate exhibited higher adhesion ability in a nidogen concentration-dependent manner, and the proliferation rate of the cells adhering to the nidogen coating at 24 hours and 48 hours was significantly higher than cells adhering to gelatin or collagen coating.

### 2.4. Double-coating effect on nidogen protein and other extracellular matrix proteins in proliferation of keratinocytes

Nidogen is well known to combine with other components to act as a bridge in the basement membrane. The synergistic effect on the cell proliferation was analyzed by double-coating with the nidogen protein and other extracellular matrix proteins.

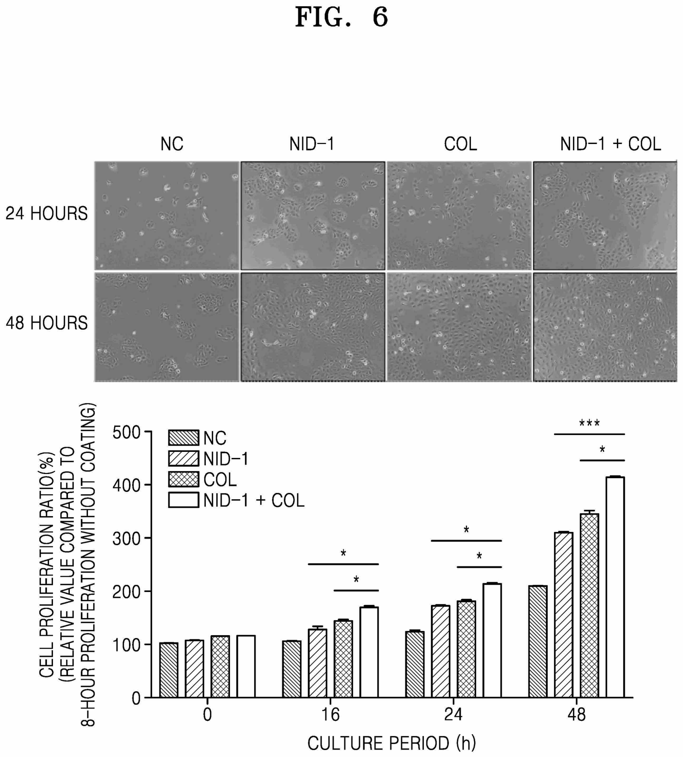

By a combination of nidogen-1 protein (10, 50 µg/mL) and collagen solution (12.5 %, 25 %, 50 %, 100 %) or a combination of nidogen-1 protein (1, 5, 10 µg/mL) and collagen solution (25 %), a culture plate (24-well plate) was coated at 4°C for 24 hours. After blocking with 0.3 % BSA solution for 1 hour, unblocked cells were washed off. The HaCaT cells were suspended in serum-free DMEM and seeded at 1 x 10⁴ cells per well. The cells were then cultured for 4 hours at 37 °C in a CO₂ incubator to allow adhesion to the dishes. After 4 hours, non-adherent cells were removed, and adherent cells were cultured for an additional 8 hours, 16 hours, 24 hours, and 48 hours. Cell images were obtained at each time point, and treated with EZ-Cytox to measure cell survival and proliferation, and the results are shown in FIG. 6.

FIG. 6 shows an image (top) and a graph (bottom) of the cell proliferation, showing a synergistic effect of the double-coating of nidogen and collagen on the cell proliferation.

As shown in FIG. 6, both the nidogen protein and collagen solution coatings demonstrated better cell adhesion and proliferation induction effects compared to the uncoated culture plate. However, the highest cell proliferation was induced in a plate coated with a combination of 10 µg/mL of the nidogen protein and the 25 % collagen solution.

Accordingly, the synergistic effect of nidogen and collagen coating on the cell proliferation was confirmed.

### Experimental Example 3. Effect of nidogen coating on ultraviolet B (UVB)-treated full-thickness artificial skin model

### 3.1. Epidermal-dermal adhesion effect by nidogen coating

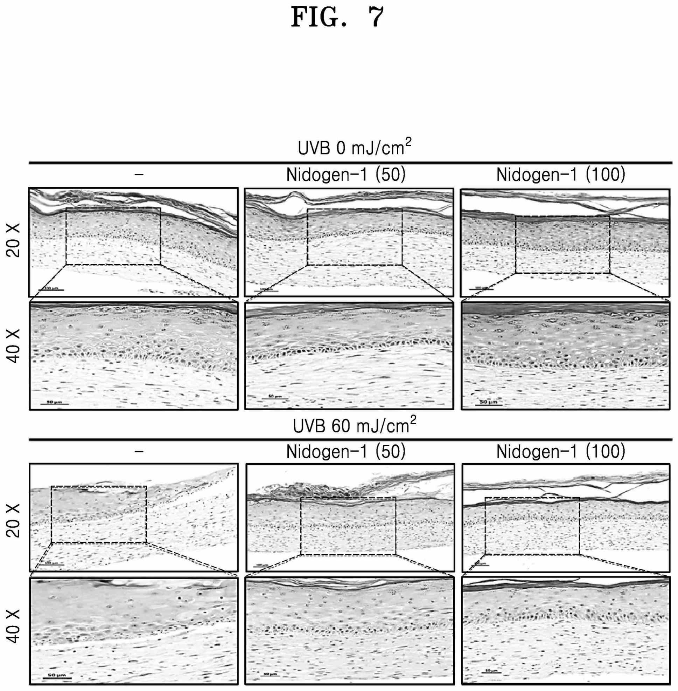

A full-thickness artificial skin model was prepared based on the method described in the literature [Larouche et al. BioResearch Open Access (2016) 5(1):320-329]. Human dermal fibroblasts (HDFs) were cultured in DMEM medium supplemented with 10 % FBS and 10 mg/mL of ascorbic acid for 4 weeks to form a dermal sheet. 50 µg/ml of nidogen-1 protein and 100 µg/ml of nidogen-1 protein were sprayed onto the dermal sheet, and the resulting dermal sheet was air-dried at room temperature for 45 minutes and washed off twice with DPBS. HEK cells were seeded on the dermal sheet and cultured for 1 week to form a dermal-epidermal structure. The formed dermal-epidermal structure was placed on another dermal sheet and exposed to air for 14 days to induce epidermis formation. During 14 days of air exposure period, the medium was replaced every two days. UVB (60 mJ/cm²) treatment was performed once on the full-thickness artificial skin model at the end of 14 days of differentiation, and the artificial skin was collected 24 hours later. After fixing the processed artificial skin with 4 % paraformaldehyde, it was prepared in paraffin blocks, sectioned into a 10 µm-thick slice, and prepared as a slide specimen for various tissue staining techniques. The fixed slide specimen was placed in a staining jar and immersed in xylene for 5 minutes to remove paraffin. Then, it was sequentially immersed in 100 %, 95 %, 90 %, 80 %, and 70 % ethanol, followed by a washing process. Finally, the resulting specimen was stained with Harris Hematoxylin (Sigma-Aldrich, Germany) for 10 minutes. Next, following processes of washing, treating with 0.5 % HCI, washing, treating with 0.5 % ammonia, and washing, the specimen was stained with eosin solution for 2 minutes. After washing, the specimen was sequentially treated with 70 %, 80 %, 95 %, and 100 % ethanol for dehydration, immersed in xylene for 1 minute, and finally mounted with Canada balsam for observation under an optical microscope. The results are shown in FIG. 7.

FIG. 7 is an image showing the epidermal-dermal adhesion effect of nidogen in the artificial skin irradiated with UVB (20X Scale bar: 100 µm, 40X Scale bar: 50 µm).

As shown in FIG. 7, under normal conditions, no significant changes were observed in the presence or absence of the nidogen protein coating. However, under UVB irradiation conditions, the full-thickness artificial skin model coated with the nidogen protein exhibited significant reduction in the epidermal-dermal separation and blistering caused by UVB irradiation compared to the uncoated artificial skin.

### 3.2. Effect of nidogen coating on restoring basement membrane structure

The effect of restoring the collapsed basement membrane (BM) structure upon UVB irradiation after nidogen coating between the epidermis and dermis of a full-thickness artificial skin model was analyzed using transmission electron microscopy (TEM).

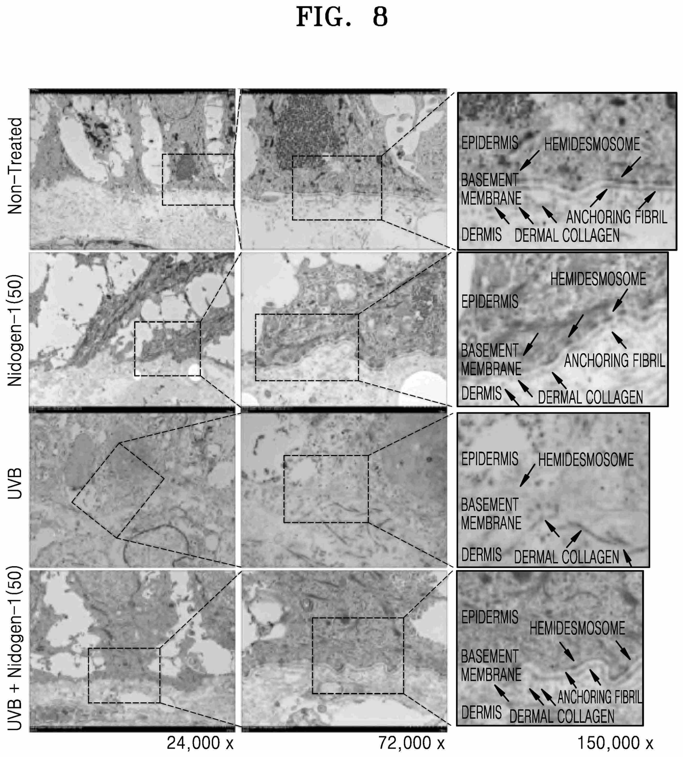

Specifically, a full-thickness artificial skin model was prepared and UVB was irradiated thereto in the same manner as in Experimental Example 3.1, so as to prepare a specimen for TEM. The specimen for TEM was fixed with 0.1 M phosphate buffer (pH 7.4) containing 2 % glutaraldehyde-2 % paraformaldehyde for 12 hours, washed with 0.1 M phosphate buffer, and then fixed with 0.1 M phosphate buffer containing 1 % OsO₄ for 2 hours. The specimen was dehydrated in an ascending ethanol series (50, 60, 70, 80, 90, 95, 100, 100 %) for 10 minutes each, and then immersed in propylene oxide for 10 minutes. The resulting specimen was polymerized in an electron microscope oven (TD-700, DOSAKA, Japan), at 65 °C for 12 hours using a Poly/Bed 812 kit (Polysciences), cut into a 200 nm-semi-thick section using a diamond knife mounted on an ultra microtome (UC7, Leica Microsystems Ltd, Vienna, Austria), stained with toluidine blue, and observed under an optical microscope. Then, the region of interest was cut into a 80 nm-thick thin section using an ultra-microtome, placed on a copper grid, and double-stained with 5 % uranyl acetate for 20 minutes and 3 % lead citrate for 7 minutes. Then, the section was photographed with a transmission electron microscope (HT7800, HITACHI, Tokyo, Japan) equipped with an RC camera at an accelerating voltage of 80 kV, and the results are shown in FIG. 8.

FIG. 8 is an image showing the restorative effect of nidogen on the BM structure in the artificial skin irradiated with UVB.

As shown in FIG. 8, in both the nidogen-non-treated group without UVB irradiation (normal group) and the nidogen-treated (coated) group, the epidermal-dermal junction or the BM was clearly observed, and the structures of hemidesmosomes, anchoring fibrils, and dermal collagen were well observed around the BM. In contrast, in the UVB-irradiated nidogen-non-treated artificial skin, the epidermal-dermal junction where the BM is located was hardly observed, and collagen with black dots was observed in the dermis. However, in the nidogen-treated group, even when UVB was irradiated, the BM and its surrounding structures were clearly observed as in the normal group, confirming the restorative effect of nidogen on UVB-damaged BM structures. Melasma skin exhibits a series of structural and functional changes in the epidermis, BM, and upper dermis, which interact to induce and maintain a localized hypermelanin production phenotype. In melasma skin, disruption or gaps in the BM were observed more frequently. That is, damage to the BM is associated with melanogenesis in melasma skin and photoaged skin.

Referring to the results above, by maintaining the BM structure and inducing binding between the BM and the extracellular matrix, the nidogen of the present invention may suppress melanin production in melasma skin or photoaging-induced skin, improve hyperpigmentation including freckles and/or blemishes, and/or induce a skin whitening effect.

### 3.3. Restorative effect of nidogen coating on epidermal differentiation

The restorative effect on the suppressed epidermal differentiation upon UVB irradiation after nidogen coating between the epidermis and dermis of a full-thickness artificial skin model was analyzed using filaggrin (FLG) staining.

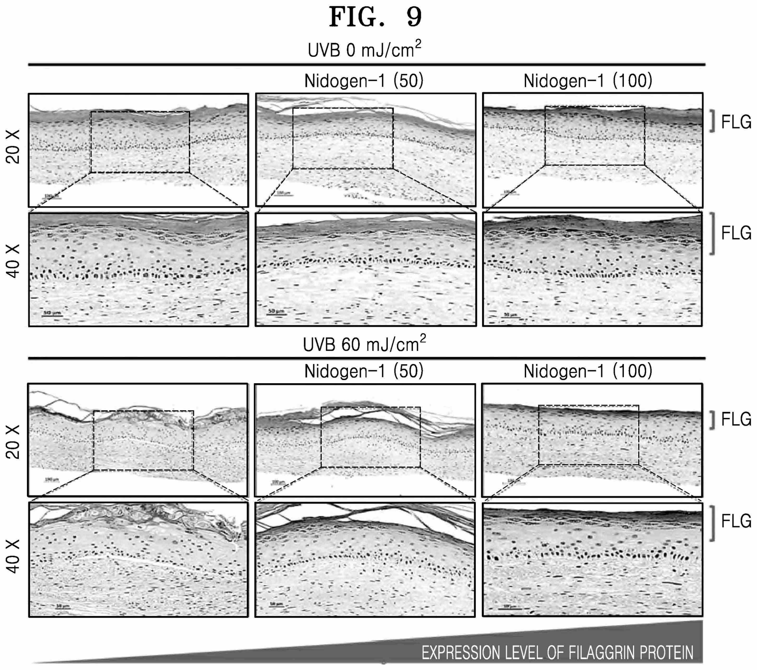

Specifically, a full-thickness artificial skin model was prepared and UVB was irradiated thereto in the same manner as in Experimental Example 3.1, and a fixed slide specimen thus prepared was washed with tap water to remove paraffin and for rehydration. Following a washing process for 3 to 5 minutes, the specimen was washed with 0.01 mol citric acid buffer solution (pH 6.0) and heated in a microwave for 5 minutes three times. The specimen was placed in phosphate buffered saline (PBS) for 5 minutes, then left in 0.3 % H₂O₂-methanol solution for 10 minutes, and finally washed with PBS for 10 minutes. The specimen was treated with blocking solution (1 % BSA) for 1 hour and then reacted with a primary antibody (anti-FLG antibody) at 4 °C overnight. After washing with PBS for 10 minutes and reacting with a secondary antibody for 1 hour, the specimen was stained with horseradish peroxidase-diaminobenzidine (HRP-DAB) and then washed with PBS for 10 minutes. Using a DAB substrate kit (Abcam, England), the specimen was reacted for about 1 minute, washed, and stained with Harris hematoxylin. After washing, the specimen was sequentially treated with 70 %, 80 %, 90 %, 95 %, and 100 % ethanol for dehydration, immersed in xylene three times at three-minute intervals, and finally mounted with Canada balsam for observation under an optical microscope. The nidogen-non-treated group, the nidogen-treated group (50 µg/ml), and the nidogen-treated group (100 µg/ml) were each stained with FLG, and the expression level of FLG therein was analyzed. The results are shown in FIG. 9.

FIG. 9 is an image showing the restorative effect of nidogen on suppressed epidermal differentiation in artificial skin irradiated with UVB (20X Scale bar: 100 µm, 40X Scale bar: 50 µm).

As shown in FIG. 9, when comparing the UVB-irradiated group and the non-irradiated group among the nidogen-non-treated group, it was observed that UVB irradiation suppressed the epidermal differentiation and the staining intensity of FLG weakened from the granular layer. In the UVB-irradiated group, compared to the nidogen-non-treated group, the suppressed epidermal differentiation phenomenon in the nidogen-treated group was overcome, and the staining intensity of FLG increased again from the granular layer. Such effects of overcoming the suppressed epidermal differentiation and inducing FLG expression were found to increase in a concentration-dependent manner with nidogen treatment (0, 50, 100 µg/mL), regardless of UVB irradiation.

Accordingly, the restorative effect of nidogen on the epidermal differentiation that has been damaged by UVB was confirmed.

### 3.4. Restorative effect of nidogen coating on proliferation of basal layer of epidermis

The recovery effect on the suppressed proliferation of the basal layer of the epidermis upon UVB irradiation after nidogen coating between the epidermis and dermis of a full-thickness artificial skin model was analyzed using Ki67 staining as a cell proliferation marker and a stemness marker.

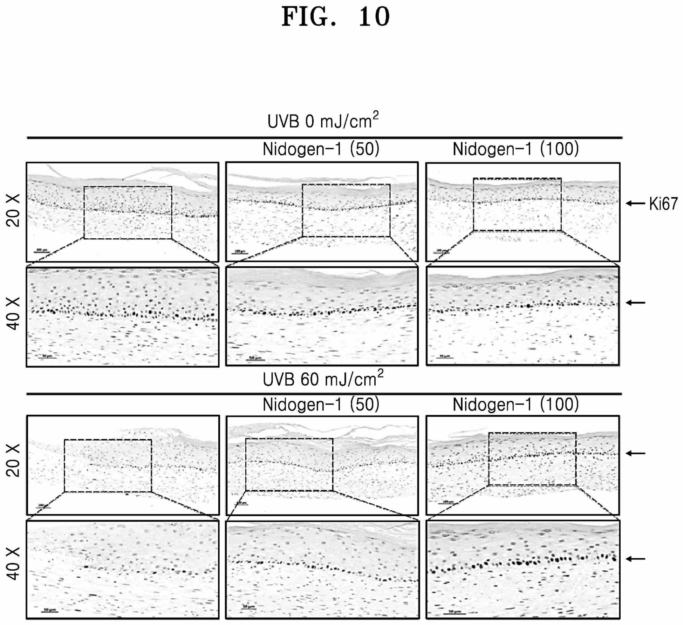

Specifically, a full-thickness artificial skin model was prepared and UVB was irradiated thereto in the same manner as in Experimental Example 3.1, and Ki67 was stained in the same manner as in Experiment 3.3 for each of the following groups: the nidogen-non-treated group, the nidogen-treated group (50 µg/ml), and the nidogen-treated group (100 µg/ml), and the expression levels were analyzed. The results are shown in FIG. 10.

FIG. 10 is an image showing the restorative effect of nidogen on the proliferation of the basal layer of the epidermis in the artificial skin irradiated with UVB (20X Scale bar: 100 µm, 40X Scale bar: 50 µm).

As shown in FIG. 10, when comparing the UVB-irradiated group and the non-irradiated group among the nidogen-non-treated group, it was observed that UVB irradiation reduced the number of Ki67 expression-positive cells in the basal layer of the epidermis. Among the UVB-irradiated groups, the number of Ki67 expression-positive cells in the basal layer of the epidermis increased again in a nidogen concentration-dependent manner in the nidogen-treated group compared to the nidogen-non-treated group.

Accordingly, the restorative effect of nidogen on the proliferation of the basal layer of the epidermis that has been damaged by UVB was confirmed.

### 3.5. Restorative effect of nidogen coating on collagen production in dermis

The restorative effect on collagen production in the dermis upon UVB irradiation after nidogen coating between the epidermis and the dermis of a full-thickness artificial skin model was analyzed using Masson's Trichrome (MT) staining.

Specifically, a full-thickness artificial skin model was prepared and UVB was irradiated thereto in the same manner as in Experiment 3.1, and a fixed slide specimens thus prepared was placed in a staining jar, deparaffinized in xylene for 4 minutes, sequentially immersed in 100 %, 95 %, 90 %, 80 %, and 70 % ethanol for hydration, and then immersed in Bouin's solution (Sigma-Aldrich, Germany) heated at to 60°C for 45 minutes. The slide specimen was washed with running tap water until it turns yellow, stained with Weigert's hematoxylin for 8 minutes to stain the nuclei, and then washed again. Next, the specimen was stained with an anionic dye (Biebrich scarlet) to stain the cytoplasm, washed, treated with phosphomolybdic acid solution, treated additionally with a color fixer for 10 minutes, and immediately stained with aniline blue solution for 5 minutes. Finally, the specimen was treated with 1 % acetic acid solution for 1 minute, washed, and sequentially immersed in 70 %, 80 %, 95 %, and 100 % ethanol for dehydration. The resulting slide was immersed in xylene for 1 minute and finally mounted with Canada balsam for observation under an optical microscope. The nidogen-non-treated group, the nidogen-treated group (50 µg/ml), and the nidogen-treated group (100 µg/ml) were each subjected to MT staining to analyze the collagen production level.

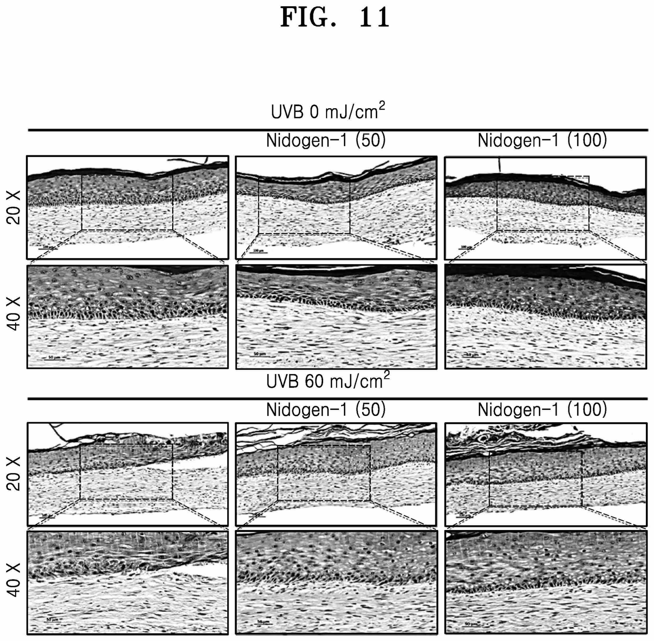

FIG. 11 is an image showing the restorative effect of nidogen on the collagen production in the dermis of artificial skin irradiated with UVB (20X Scale bar: 100 µm, 40X Scale bar: 50 µm).

As shown in FIG. 11, when comparing the UVB-irradiated group and the non-irradiated group among the nidogen-non-treated group, it was observed that UVB irradiation significantly reduced the collagen production in the dermis. Among the UVB-irradiated groups, the collagen production in the dermis was remarkably increased in the nidogen-treated group compared to the nidogen-non-treated group, in a tendency to increase in a nidogen concentration-dependent manner.

Accordingly, the restorative effect of nidogen on the collagen production in the dermis that has been damaged by UVB was confirmed.

### Experimental Example 4. Effect of nidogen coating on induction of stemness marker expression

The expression of stemness markers, Ki67 and Keratin 14 (KRT14) was measured by Western blotting in human epidermal keratinocytes (HEKs), upon nidogen coating in a culture dish.

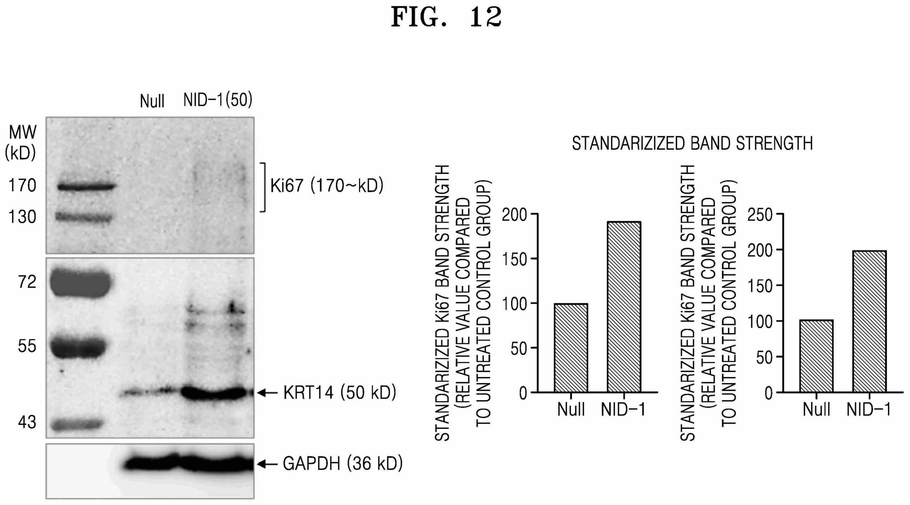

HEKs that were cultured for 24 hours on a plate coated with nidogen (50 µg/ml) in a culture dish were obtained, and the obtained cells were lysed. Proteins contained in the cell lysate were quantified, and for each test group and a control group, 8 µg of the proteins were separated by 7 % polyacrylamide gel electrophoresis (sodium dodecyl sulfate-polyacrylamide gel electrophoresis: SDS-PAGE). The separated proteins were then transferred to a polyvinylidene fluoride (PVDF) membrane, semi-dried for 50 minutes, and blocked with 5 % skim milk for 1 hour. Afterwards, the PVDF membranes were each reacted overnight at 4 °C with primary antibodies, such as anti-cytokeratin 14 (ab9220, 1:500) and anti-Ki67 (MA5-14520, 1:500), and anti-GAPDH (sc47724, 1:2000). Next day, the PVDF membrane was washed with TBST solution containing Tween, Tris, and NaCl, and reacted with secondary antibodies conjugated with anti-mouse IgG and anti-rabbit HRP at room temperature for 1 hour. The protein bands were analyzed using an Amersham imager 680, and the results are shown in FIG. 12.

FIG. 12 shows a western blot image (left) showing an increase in the expression of Ki67 and KRT14, which are proteins related to the stemness of cells upon the nidogen coating, and a graph (right) quantifying the intensity of each band after standardization.

As shown in FIG. 12, the expression of GAPDH was clearly observed in both the nidogen-non-treated group (Null) and the nidogen-treated group (50 µg/ml), whereas the expression of Ki67 and KRT14 proteins, which are markers of the stemness of HEKs, was more clearly observed in the nidogen-treated group (50 µg/ml) group than in the nidogen-non-treated group. Accordingly, it was confirmed that the nidogen coating has induced the expression of proteins related to the maintenance of the stemness of keratinocytes.

### Experimental Example 5. Synergistic effect of nidogen coating and EPC-CM treatment

The resistance effect of EPC-CM against oxidative stress has been well characterized in human fibroblasts (Dermatol Ther (Heidelb). 2018 Jun;8(2):229-244). Accordingly, it was aimed to investigate the antioxidant effects of EPC-CM on HaCaT cells, which are HEKs, and the synergistic effects of EPC-CM and nidogen on keratinocytes.

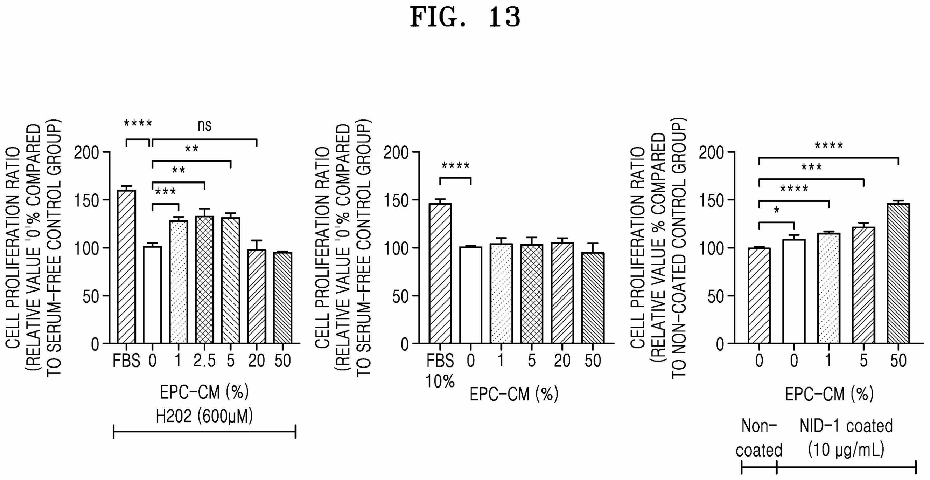

The HaCaT cells were suspended in DMEM containing 10 % FBS, and seeded at 3 x 10⁴ cells per well and cultured at 37 °C in a CO₂ incubator for one day. Next day, after washing with DPBS, the medium was replaced with serum-free DMEM containing EPC-CM diluted to final concentrations of 0, 1, 2.5, 5, 20, and 50 % (v/v), and the cells were cultured at 37 °C in a CO₂ incubator for 24 hours. H₂O₂ at a concentration of 600 µM was treated with the same medium, and after an additional 24 hours of culturing, EZ-Cytox was used to measure the cell proliferation, and the results are shown in FIG. 13 (left).

In comparison, it was also aimed to determine whether there was a difference between the EPC-CM treatment alone and the EPC-CM treatment after nidogen-1 protein coating under normal conditions. The HaCaT cells were suspended in DMEM containing 10 % FBS, and seeded at 3 x 10⁴ cells per well and cultured at 37 °C in a CO₂ incubator for one day. Next day, after washing with DPBS, the medium was replaced with serum-free DMEM containing EPC-CM diluted to final concentrations of 0, 1, 5, 20, and 50 % (v/v), and the cells were cultured at 37 °C in a CO₂ incubator for 24 hours. Afterwards, the medium was replaced with serum-free DMEM, and EZ-Cytox was used to measure the cell proliferation, and the results are shown in FIG. 13 (center).

Then, it was confirmed whether there was a difference in the effect of the EPC-CM depending on the presence or absence of nidogen-1 protein under normal conditions. Nidogen-1 quantified by the BCA assay method was diluted to a concentration of 10 µg/ml using DPBS, dispensed into a non-treated cell culture plate (24-well plate) at 500 µI per well, and then allowed for coating at 4 °C for one day (control: 500 µI of DPBS dispensed). Next day, 500 µl of 0.5 % BSA solution was dispensed into each well, followed by incubation at room temperature for 1 hour and washing with DPBS. Prepared HaCaT cells were suspended in serum-free DMEM, seeded at 3 x 10⁴ cells per well, and cultured for one day at 37 °C in a CO₂ incubator. Next day, the medium was replaced with serum-free DMEM containing EPC-CM diluted to final concentrations of 0, 1, 5, and 50 % (v/v), and the cells were cultured at 37 °C in a CO₂ incubator for 24 hours. After 24 hours, cell images were obtained, the medium was replaced by serum-free DMEM, and EZ-Cytox was used to measure the cell proliferation. The results are shown in FIG. 13 (right).

FIG. 13 is a graph showing the synergistic effect of the nidogen coating and the EPC-CM on the cell proliferation.

As shown in FIG. 13, it was confirmed that the restorative effect on the cell proliferation exhibited even with low concentrations (up to 5 %) of EPC-CM treatment under oxidative stress conditions that have been induced by H₂O₂. Unlike under oxidative stress conditions, no significant restorative effect on the cell proliferation was observed regardless of the EPC-CM treatment concentration under normal conditions. However, depending on the nidogen coating, the cell proliferation increased significantly in a concentration-dependent manner with the EPC-CM treatment.

Accordingly, the synergistic effect of the nidogen coating and the EPC-CM on the cell proliferation was confirmed.

### Experimental Example 6. Cellular and clinical effects of products comprising nidogen protein and EPC-CM

A formulation containing 33 % EPC-CM as a main ingredient and a formulation containing 33 % EPC-CM and 10 ppm (10 µg/mL) of nidogen-1 protein as main ingredients were each prepared, and effects of the formulations on the proliferation of HDFs were evaluated.

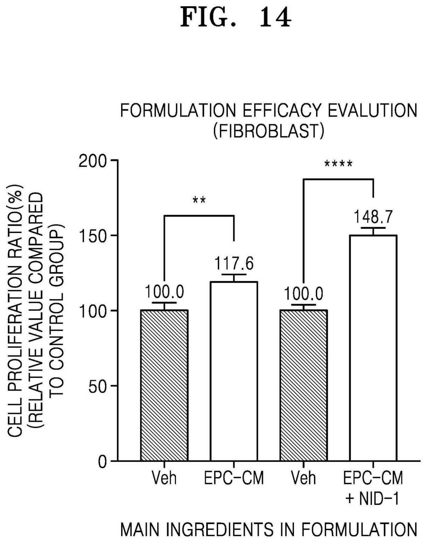

A dilution concentration at which no toxicity was observed was set by a vehicle control which includes an excipient used in the manufacture of the formulation (25 %). 1 x 10⁴ of HDFs were seeded into a 48-well plate and cultured for 24 hours. Next day, the medium was replaced with serum-free DMEM and culture for an additional 24 hours. Each formulation was diluted to 25 % with serum-free DMEM, treated with the cells for 24 hours, and evaluated for the cell survival and proliferation using EZ-Cytox. The results are shown in FIG. 14.

FIG. 14 is a graph showing the effect of the formulation comprising nidogen and the EPC-CM on the cell proliferation.

As shown in FIG. 14, the formulation comprising EPC-CM significantly increased the cell proliferation compared to the vehicle control including an excipient only. In comparison, it was confirmed that the formulation comprising both EPC-CM and nidogen exhibited a greater effect.

### Experimental Example 7. Nidogen coating and effect thereof on cell wound healing

The wound healing effect of wild-type nidogen-1 protein was evaluated using cell scratch assay.

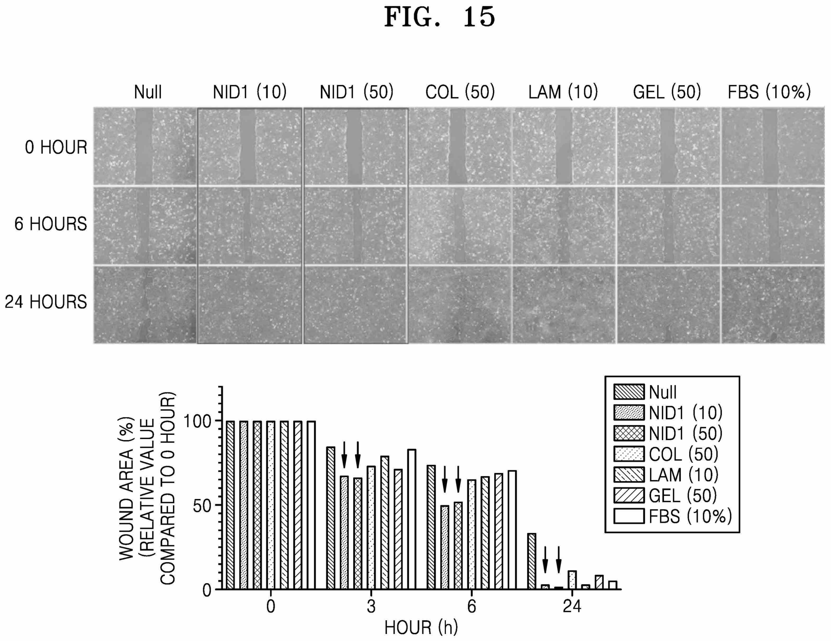

Specifically, nidogen (NID1) (10, 50 mg/mL), collagen (COL) (50 mg/mL), laminin (LAM) (10 mg/mL), and gelatin (GEL) (50 mg/mL) were each added to a 6-well culture dish and allowed for coating, followed by blocking with 0.3 % BSA for 1 hour. Then, HaCaT cells were seeded at 1x10⁵ per well and washed off after 3 hours. After growing the cells to 100 % confluence, a scratch was made using a 200 mL tip. Afterwards, the medium was replaced with serum-free medium or 10 % FBS medium (positive control), and images were captured using an optical microscope after 3, 6, and 24 hours. The captured images were measured using Image J software to determine the lesion area, and the results are shown in FIG. 15.

As shown in FIG. 15, it was confirmed that the wound healing effect on keratinocytes was significantly greater when coated with nidogen than when coated with collagen, laminin, or gelatin.

Referring to the results above, it was confirmed that nidogen exhibited effects on the cell adhesion and proliferation in a concentration-dependent manner, and when used in combination with EPC-CM or collagen, it exhibited synergistic effects on the cell proliferation. Accordingly, nidogen which affects the epidermis and the dermis through strengthening of the BM, may be used for skin improvement including skin damage recovery, skin barrier strengthening, suppression or prevention of hyperpigmentation, moisturization, skin elasticity, and anti-aging.

The foregoing descriptions are only for illustrating the present disclosure, and it will be apparent to a person having ordinary skill in the art to which the present invention pertains that the embodiments disclosed herein can be easily modified into other specific forms without changing the technical spirit or essential features. Therefore, it should be understood that Examples described herein are illustrative in all respects and are not limited.

## Claims

1. A cosmetic composition for skin improvement, comprising a protein, nidogen, as an active ingredient.

2. The cosmetic composition for skin improvement according to claim 1, further comprising an epidermal progenitor cell-conditioned medium.

3. The cosmetic composition for skin improvement according to claim 1, wherein the protein has an amino acid sequence of SEQ ID NO: 1.

4. The cosmetic composition for skin improvement according to claim 1, wherein the skin improvement comprises one or more selected from the group consisting of skin barrier improvement, prevention or amelioration of hyperpigmentation, prevention or amelioration of skin wrinkles, skin moisturization, skin anti-aging enhancement, skin defense enhancement, skin elasticity enhancement, skin soothing, skin regeneration, and prevention or amelioration of skin diseases.

5. The cosmetic composition for skin improvement according to claim 1, wherein the skin improvement comprises the following characteristics:
- inhibition of epidermal-dermal separation by UVB or ultraviolet rays;
- maintenance of a basement membrane structure or keratinocyte arrangement in a basal layer, by UVB or ultraviolet rays; or
- alleviation of inhibition of epidermal differentiation, by UVB or ultraviolet rays.

6. The cosmetic composition for skin improvement according to claim 1, wherein the protein improves binding between a basement membrane and an extracellular matrix, or density of the dermis.

7. The cosmetic composition for skin improvement according to claim 1, wherein the protein promotes production of intracellular collagen, Ki67 or filaggrin.

8. A cosmetic composition for maintenance of stemness of the epidermis, comprising a protein, nidogen, as an active ingredient.

9. The cosmetic composition for maintenance of stemness of the epidermis according to claim 8, wherein the protein upregulates expression of genes of the group consisting of Ki67, keratin 14 (KRT14), integrin alpha-6 (ITGA6), TP63, and SRY-box transcription factor 2 (SOX2).

10. The cosmetic composition for maintenance of stemness of the epidermis according to claim 8, wherein the maintenance of stemness comprises one or more selected from the group consisting of inhibition of cell aging of stem cells, increase in cell proliferation ability, increase in functionality of stem cells, increase in telomerase activity, increase in expression of stem cell-related factors, increase in protein homeostasis, and increase in cell migration activity.

11. A pharmaceutical composition for prevention, amelioration, or treatment of skin damage, comprising a nidogen protein as an active ingredient.

12. The pharmaceutical composition according to claim 11, wherein the skin damage comprises a skin wound, a skin scar, or a skin burn.

13. A composition for external preparation on skin for skin improvement or prevention, amelioration, or treatment of skin damage, comprising a nidogen protein as an active ingredient.

14. A medium composition for maintenance of stemness of the epidermis, comprising a nidogen protein as an active ingredient.

15. Artificial skin comprising: a dermal layer including a fibroblast; and an epidermal layer including a keratinocyte and a nidogen protein.

16. The artificial skin according to claim 15, wherein the nidogen protein is present in a basement membrane of the epidermal layer.

17. The artificial skin according to claim 15, wherein the nidogen protein has an amino acid sequence of SEQ ID NO: 1.

18. A preparation method for artificial skin, comprising:
preparing a dermal layer by culturing a fibroblast;
applying a nidogen protein to the dermal layer; and
preparing an epidermal layer by applying a keratinocyte to the dermal layer on which the nidogen protein has been applied.

19. The preparation method according to claim 18, wherein the preparing of the epidermal layer comprises: manufacturing a dermal-epidermal structure from applied keratinocyte; and then culturing the dermal-epidermal structure on a separate dermal layer.

20. A method of maintaining stemness of an epidermis of skin cells, comprising culturing skin cells in a medium comprising isolated nidogen protein.

21. A method of improving a skin condition or preventing, ameliorating, or treating skin damage, the method comprising administering an effective amount of a nidogen protein to a subject in need thereof.

22. Use of a nidogen protein for the manufacture of a preparation for improvement of a skin condition or prevention, amelioration, or treatment of skin damage.
